# EUROPEAN PATENT SPECIFICATION

(11) **EP 2 991 715 B1**
(45) Date of publication and mention of the grant of the patent: **10.06.2020**
(21) Application number: 14720259.2
(22) Date of filing: 03.04.2014
(51) Int. Cl.: A61M 16/00

(54) **CRITICAL CARE VENTILATOR WITH MOUTH PIECE VENTILATION**
VENTILATOR FÜR DIE INTENSIVSTATION MIT MUNDSTÜCKBELÜFTUNG
VENTILATEUR DE SOINS CRITIQUES AVEC VENTILATION PAR PIÈCE BUCCALE

(30) Priority: 03.04.2013 US 201361807974 P
(43) Date of publication of application: 09.03.2016
(73) Proprietor: Koninklijke Philips N.V., 5656 AG Eindhoven (NL)
(72) Inventor: TRUSCHEL, William, Anthony, NL-5656 AE Eindhoven (NL); BERRY ANN, Nathan, John, NL-5656 AE Eindhoven (NL)
(74) Representative: Schudelaro, Antonius Adrianus Petrus
(86) International application number: PCT/IB2014/060393
(87) International publication number: WO 2014/162283

(56) References cited:
- EP-A2- 0 714 670
- US-A1- 2010 071 689
- US-A1- 2010 071 696

## Description

This patent application claims the priority benefit under 35 U.S.C. § 119(e) of U.S. Provisional Application No. 61/807,974 filed on April 3, 2013.

### BACKGROUND OF THE INVENTION

### 1. Field of the Invention

The present disclosure pertains to systems and methods used for providing respiratory support to a subject, in particular, by intermittently delivering pressurized flow of breathable gas to an airway of the subject.

### 2. Description of the Related Art

Use of non-invasive respiratory support for patients with chronic respiratory failure may eliminate or delay the need for tracheostomy and may provide quality of life benefits such as fewer pulmonary infections, easier vocalization, and improved swallowing ability. One possible method of providing non-invasive respiratory support is to allow the patient to access ventilation as needed and initiate a breath from a ventilator. A typical ventilator may not be used for providing such respiratory support because nuisance alarms triggered by the intermittent nature of such a method. US2010/071689A1 describes a ventilator with a safe standby mode. The safe standby mode allows a user to disconnect a patient from the ventilator, without the ventilator generating alarms and while maintaining previously entered ventilation parameters. In addition, while in the safe standby mode, a patient connection status is monitored, and a ventilation mode is entered automatically if the ventilator determines that a patient is connected to the ventilator while the ventilator is in the safe standby mode. EP0714670A2 describes improved methodology and systems for delivery of breathing gas such as for the treatment of obstructive sleep apnea through application of alternating high and low level positive airway pressure within the airway of the patient with the high and low airway pressure being coordinated with the spontaneous respiration of the patient, and improved methods and apparatus for triggering and for leak management in such systems.

### SUMMARY OF THE INVENTION

In an aspect, there is provided a ventilation system configured to provide respiratory support by intermittently delivering pressurized flow of breathable gas to a subject as defined in appended claim 1.

Accordingly, one or more embodiments provide a ventilation system configured to provide respiratory support by intermittently delivering pressurized flow of breathable gas to a subject. The ventilation system includes a pressure generator, an interface appliance, one or more sensors, and one or more processors configured to execute one or more computer program modules. The pressure generator is be configured to generate pressurized flow of breathable gas for delivery to an airway of the subject. The interface appliance is configured to be at least partially and releasably received into an airway orifice of the subject and further configured to deliver the pressurized flow of breathable gas generated by the pressure generator to the airway of the subject. The one or more sensors are configured to generate output signals conveying information indicating whether the subject is ready to receive pressurized flow of breathable gas through the interface appliance. The computer program modules include a delivery triggering module, and control module. The delivery triggering module is configured to determine whether the subject is ready to receive the pressurized flow of breathable gas. The control module is configured to initiate and/or terminate delivery of the pressurized flow of breathable gas to the airway of the subject through the interface appliance based on determinations by the delivery triggering module as to whether the subject is ready to receive the pressurized flow of breathable gas. The control module is further configured to control the pressure generator to adjust one or more parameters of the pressurized flow of breathable gas based on a prescribed therapy regimen designed to ventilate the subject.

It is yet another aspect of one or more examples to provide method of providing respiratory support to a subject using a ventilation system having a pressure generator, an interface appliance, one or more sensors, and one or more processors configured to execute one or more computer program modules. The method includes determining, based on output signals from the one or more sensors, whether the subject is ready to receive pressurized flow of breathable gas through the interface appliance generated by the pressure generator, and delivering pressurized flow of breathable gas to an airway of the subject, as prescribed by a therapy regimen designed to ventilate the subject.

It is yet another aspect of one or more embodiments to provide a system configured to provide respiratory support by intermittently delivering pressurized flow of breathable gas to a subject. The system includes means for generating pressurized flow of breathable gas for delivery to an airway of the subject, means for delivering the pressurized flow of breathable gas to the airway of the subject, means for generating output signals conveying information indicating whether the subject is ready to receive pressurized flow of breathable gas through means for delivering the pressurized flow of breathable gas, means for determining whether the subject is ready to receive the pressurized flow of breathable gas, means for initiating and/or terminating delivery of the pressurized flow of breathable gas to the airway of the subject, and means for controlling the means for generating pressurized flow of breathable gas to adjust one or more parameters of the pressurized flow of breathable gas based on a prescribed therapy regimen designed to ventilate the subject. The means for delivering the pressurized flow of breathable gas is configured to be at least partially and releasably received into an airway orifice of the subject. The initiation and/or termination of delivery of the pressurized flow of breathable gas are based on determination as to whether the subject is ready to receive the pressurized flow of breathable gas.

These and other aspects, features, and characteristics of the present disclosure, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of any limits.

### BRIEF DESCRIPTION OF THE DRAWINGS

FIG. 1 schematically illustrates a ventilation system to provide respiratory support by intermittently delivering pressurized flow of breathable gas to a subject; and
FIG. 2 illustrates a method of providing respiratory support to a subject according to the principles of the present invention.

### DETAILED DESCRIPTION OF EXEMPLARY EMBODIMENTS

As used herein, the singular form of "a", "an", and "the" include plural references unless the context clearly dictates otherwise. As used herein, the statement that two or more parts or components are "coupled" shall mean that the parts are joined or operate together either directly or indirectly, i.e., through one or more intermediate parts or components, so long as a link occurs. As used herein, "directly coupled" means that two elements are directly in contact with each other. As used herein, "fixedly coupled" or "fixed" means that two components are coupled so as to move as one while maintaining a constant orientation relative to each other.

As used herein, the word "unitary" means a component is created as a single piece or unit. That is, a component that includes pieces that are created separately and then coupled together as a unit is not a "unitary" component or body. As employed herein, the statement that two or more parts or components "engage" one another shall mean that the parts come in contact with one another either directly or through one or more intermediate parts or components. As employed herein, the term "number" shall mean one or an integer greater than one (i.e., a plurality).

Directional phrases used herein, such as, for example and without limitation, top, bottom, left, right, upper, lower, front, back, and derivatives thereof, relate to the orientation of the elements shown in the drawings and are not limiting upon the claims unless expressly recited therein.

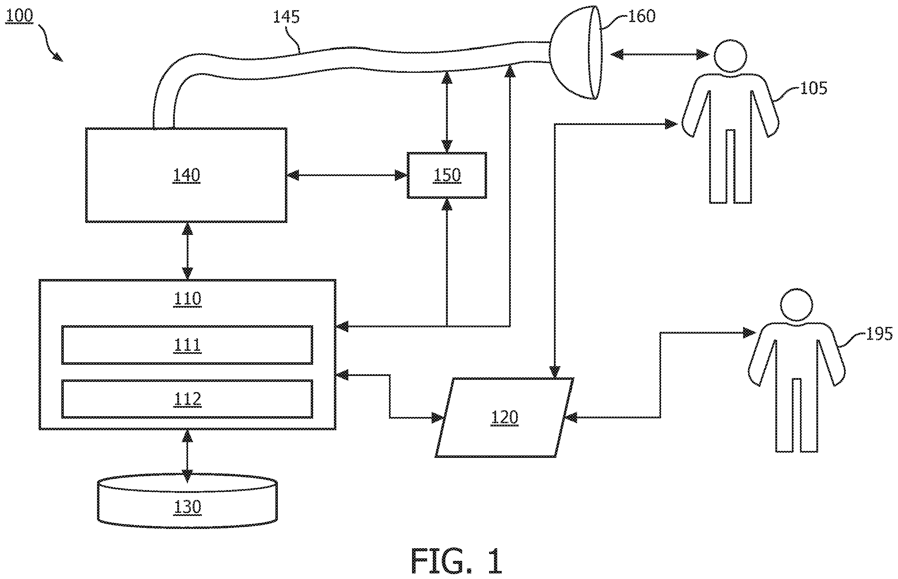

FIG. 1 schematically illustrates a ventilation system 100 configured to provide respiratory support by intermittently delivering pressurized flow of breathable gas to a subject 105. Ventilation system 100 may be implemented as, integrated with, and/or operating in conjunction with a respiratory device that provides a flow of breathable gas along a flow path to subject 105.

System 100 may include one or more of a pressure generator 140, an interface appliance 160, one or more sensors 150, and a processor 110. Processor 110 may be configured to execute one or more computer program modules including a delivery triggering module 111, and a control module 112 configured to control pressure generator 140 and to initiate and/or terminate delivery of pressurized flow of breathable gas to the subject 105. System 100 may additionally include an electronic storage 130, a user interface 120, and/or other components. System 100 may be configured to provide respiratory therapy to subject 105.

Pressure generator 140 of system 100 in FIG. 1 may be integrated, combined, or connected with a ventilator and configured to provide a pressurized flow of breathable gas for delivery to the airway of subject 105, e.g. via one or more interface appliances 160. Interface appliance 160 may sometimes be referred to as a delivery circuit.

Pressure generator 140 may include one or more of a bellows, a blower, a compressor, a pressurized gas source (e.g., wall gas, a Dewar, and/or other gas sources), and/or other mechanisms for pressurizing gas.

Pressure generator 140 may be integrated, combined, or connected with one or more sensors 150 configured to generate output signals conveying information indicating whether the subject 105 is ready to receive pressurized flow of breathable gas through the interface appliance 160. One or more sensors 150 may be further configured to provide information such as, for example, pressure, flow rate, temperature, composition and/or other parameters pertaining to the pressurized flow of the breathable gas.

System 100 may be configured to adjust and/or maintain levels of pressure, flow, humidity, velocity, acceleration, and/or other parameters of the humidified, pressurized flow of breathable gas. One or more adjustments may occur when subject 105 has made an inspiratory effort. In some embodiments, one or more operating levels (e.g. pressure, volume, etc.) are adjusted on a relatively ongoing manner (e.g., each breath, every few breaths, every few seconds, etc.) during an inspiratory effort. Alternatively, and/or simultaneously, adjustments to one or more operating levels of system 100 and/or any component thereof may be made more intermittently and/or between inspiratory efforts rather than during an individual inspiratory effort. One or more sensors 150 may be configured to generate output signals indicating an inspiratory effort based on particular movements made by subject 105 e.g. when the subject 105 moves his/her mouth or tongue, or when the subject has made a diaphragmatic movement resulting in a pressure drop near the airway orifice.

In the configuration depicted in FIG. 1, pressure generator 140 fluidly communicates with interface appliance 160 via a conduit 145 configured to carry the pressurized flow of breathable gas to interface appliance 160. Conduit 145 may be a hose or a pipe made from having a flexible length. In various embodiments, conduit 145 may be a made from materials that are non-reactive to breathable gases such as, for example, silicone, polyethylene, polyurethane, polypropylene, metalized polyethylene terephthalate, polyethylene coated aluminum, and so forth.

Conduit 145 fluidly communicates with pressure generator 140 to convey the pressurized flow of breathable gas to interface appliance 160. Interface appliance 160 fluidly communicates with conduit 145 to receive the pressurized flow of breathable gas and convey to an airway orifice (e.g. mouth) of subject 105. Interface appliance 160 is configured to be at least partially and releasably received into an airway orifice (e.g. mouth) of subject 105. The configuration of various components in FIG. 1 is not intended to limit the scope of the described technology in any way.

Interface appliance 160 of system 100 in FIG. 1 is configured to deliver the pressurized flow of breathable gas to subject 105, e.g. to the airway of subject 105. Interface appliance 160 may be configured to reduce and/or inhibit condensation from forming along the path of delivery of a (humidified and/or pressurized) flow of breathable gas to subject 105. In various embodiments, interface appliance 160 may be configured such that subject 105 does not exhale back into interface appliance 160. Interface appliance 160 may include other components and appliances (not shown) suitable for the described function. For example, the interface appliance 160 may include a diaphragm or barrier to prevent bodily fluids of subject 105 (e.g. saliva, mucus, etc.) from flowing into interface appliance 150, or conduit 145. In some embodiments, interface appliance 160 may be configured to be removably coupled to conduit 145.

In one embodiment, pressure generator 140 is a dedicated ventilation device and interface appliance 160 is configured to be removably coupled with another interface appliance being used to deliver respiratory therapy to subject 105. In another embodiment, interface appliance 160 may include a regulator (not shown) for controlling the maximum pressure that may be delivered via interface appliance 160. In one embodiment, interface appliance 160 is configured to engage the airway of subject 105 without an intervening appliance. In this embodiment, interface appliance 160 may include one or more of a tube, a mouthpiece, a nasal cannula, a nasal mask, a nasal/oral mask, and/or other interface appliances that communicate a flow of gas with an airway of a subject. In some embodiments, interface appliance 160 is configured to form a seal with between the airway orifice (e.g. mouth) of subject 105 and interface appliance 160. The present disclosure is not limited to these examples, and contemplates delivery of the pressurized flow of breathable gas to subject 105 using any interface appliance.

Electronic storage 130 of system 100 in FIG. 1 comprises electronic storage media that electronically stores information. The electronic storage media of electronic storage 130 may include one or both of system storage that is provided integrally (i.e., substantially non-removable) with system 100 and/or removable storage that is removably connectable to system 100 via, for example, a port (e.g., a USB port, a FireWire port, etc.) or a drive (e.g., a disk drive, etc.). Electronic storage 130 may include one or more of optically readable storage media (e.g., optical disks, etc.), magnetically readable storage media (e.g., magnetic tape, magnetic hard drive, floppy drive, etc.), electrical charge-based storage media (e.g., EPROM, EEPROM, RAM, etc.), solid-state storage media (e.g., flash drive, etc.), and/or other electronically readable storage media. Electronic storage 130 may store software algorithms, information determined by processor 110, information received via user interface 120, and/or other information that enables system 100 to function properly. For example, electronic storage 130 may record or store one or more gas and/or respiratory parameters (as discussed elsewhere herein), and/or other information. Electronic storage 130 may be a separate component within system 100, or electronic storage 130 may be provided integrally with one or more other components of system 100 (e.g., processor 110).

User interface 120 of system 100 in FIG. 1 is configured to provide an interface between system 100 and a user (e.g., user 195, who can also be subject 105, or a caregiver, or a therapy decision-maker, etc.) through which the user can provide information to and receive information from system 100. This enables data, results, and/or instructions and any other communicable items, collectively referred to as "information," to be communicated between the user and system 100. An example of information that may be conveyed to user 195 is a report detailing operational settings of interface appliance 160 as selected and/or preferred by subject 105. An example of information that user 195 or subject 105 may provide to system 100 is a target temperature or target humidity level during respiratory therapy. Examples of interface devices suitable for inclusion in user interface 120 include a keypad, buttons, switches, a keyboard, knobs, dials, levers, a display screen, a touch screen, speakers, a microphone, an indicator light, an audible alarm, and a printer. Information may be provided to user 195 or subject 105 by user interface 120 in the form of auditory signals, visual signals, tactile signals, and/or other sensory signals.

It is to be understood that other communication techniques, either hardwired or wireless, are also contemplated herein as user interface 120. For example, in one embodiment, user interface 120 may be integrated with a removable storage interface provided by electronic storage 130. In this example, information is loaded into system 100 from removable storage (e.g., a smart card, a flash drive, a removable disk, etc.) that enables the user(s) to customize the embodiment of system 100. Other exemplary input devices and techniques adapted for use with system 100 as user interface 120 include, but are not limited to, an RS-232 port, RF link, an IR link, modem (telephone, cable, Ethernet, internet or other). In short, any technique for communicating information with system 100 is contemplated as user interface 120.

One or more sensors 150 of system 100 in FIG. 1 are configured to generate output signals conveying measurements related to parameters of the flow of breathable gas within system 100. These parameters may include one or more of flow, (airway) pressure, barometric pressure, temperature, humidity, velocity, acceleration, and/or other parameters.

One or more sensors 150 may be in fluid communication with conduit 145, interface appliance 160, and/or other components of system 100. One or more sensors 150 may generate output signals related to physiological parameters pertaining to subject 105.

One or more sensors 150 may generate output signals conveying information related to parameters associated with an airway of subject 105 such as, for example, breathing rate, composition, temperature, and/or humidity of the gas delivered, delivery volume and/or pressure of the gas to the airway of subject 105, and/or a respiratory effort by subject 105. For example, a parameter may be related to a mechanical unit of measurement of a component of pressure generator 140 (or of a device that pressure generator 140 is integrated, combined, or connected with) such as valve drive current, rotor speed, motor speed, blower speed, fan speed, or a related measurement that may serve as a proxy for any of the previously listed parameters through a previously known and/or calibrated mathematical relationship. Resulting signals or information from one or more sensors 150 may be transmitted to processor 110, user interface 120, electronic storage 130, and/or other components of system 100. This transmission may be wired and/or wireless.

One or more sensors 150 may generate output signals conveying information indicating whether subject 105 is ready to receive the pressurized flow of breathable gas through interface appliance 160. For example, in an embodiment where interface appliance 160 is a mouthpiece, readiness of subject 105 is indicated when subject 105 receives interface appliance 160 in his/her mouth and/or makes an inspiratory effort. An inspiratory effort may be indicated, for example, when subject 105 moves his/her mouth and/or tongue, or performs a diaphragmatic movements resulting in a negative pressure at interface appliance 160. In various embodiments, engagement of subject 105 with interface appliance 160 may be detected using one or more sensors 150 including, for example, a push button or a touch sensor.

One or more sensors 150 may include one or more of, for example, accelerometer, positional sensor, movement sensor, pressure sensor, flow-meter, humidity sensor, carbon dioxide and/or carbon monoxide sensor, light sensor, infra-red (IR) sensor, electromagnetic sensor, electrode, tilt meter, (video) camera, touch sensors, push-button type engagement sensor, and/or other sensors.

The illustration of a sensor 150 at or near interface appliance 160 is not intended to be limiting, though that location may be preferred in some embodiments to provide feedback and/or information regarding the flow rate, pressure, volume, and other parameters of the pressurized flow of breathable gas being delivered to airway of subject 105.

Processor 110 of system 100 in FIG. 1 is configured to provide information processing capabilities in system 100. As such, processor 110 includes one or more of a digital processor, an analog processor, a digital circuit designed to process information, an analog circuit designed to process information, and/or other mechanisms for electronically processing information. Although processor 110 is shown in FIG. 1 as a single entity, this is for illustrative purposes only. In some embodiments, processor 110 includes a plurality of processing units.

As is shown in FIG. 1, processor 110 is configured to execute one or more computer program modules. The one or more computer program modules include one or more of delivery triggering module 111, control module 112, and/or other modules. Processor 110 may be configured to execute modules 111 and 112 by software; hardware; firmware; some combination of software, hardware, and/or firmware; and/or other mechanisms for configuring processing capabilities on processor 110.

It should be appreciated that although modules 111 and 112 are illustrated in FIG. 1 as being co-located within a single processing unit, in embodiments in which processor 110 includes multiple processing units, one or more of modules 111 and 112 may be located remotely from the other modules. The description of the functionality provided by the different modules 111 and 112 described herein is for illustrative purposes, and is not intended to be limiting, as any of modules 111 and 112 may provide more or less functionality than is described. Note that processor 110 may be configured to execute one or more additional modules that may perform some or all of the functionality attributed below to one of modules 111 and 112. In some embodiments, some or all of the described functionality of an individual computer program module may be incorporated, shared, embedded, and/or integrated into one or more other computer program modules or elsewhere within system 100.

Respiratory therapy may be implemented as pressure control, pressure support, volume control, and/or other types of support and/or control. For example, when subject 105 is ready to receive the pressurized flow of breathable gas, the pressure of the pressurized flow of breathable gas may be adjusted to an inspiratory pressure. Alternatively, the pressurized flow of breathable gas may be delivered to subject 105 for a particular duration so as to deliver a particular volume of breathable gas to subject 105 when the subject engages with interface appliance 160, and is ready to receive the pressurized flow of breathable gas. In some embodiments, the respiratory therapy may prescribe delivery of as little as a partial breath to the subject. The delivery may be synchronized with engagement of the subject with interface appliance 160. It is contemplated that some therapy regimens may prescribe delivery of the pressurized flow of breathable gas when the subject is merely engaged with interface appliance 160. Other schemes for providing respiratory support and/or ventilation through the delivery of the pressurized flow of breathable gas are contemplated. Subject 105 may or may not need to initiate one or more phases of respiration.

As used herein, "breath" refers to a single inhalation of breathable gas by the subject. "Breath" is also synonymously used as a volume of breathable gas comprised in a single inhalation by the subject.

Delivery triggering module 111 may be configured to determine whether the subject is ready to receive the pressurized flow of breathable gas. Delivery triggering module 111 may be configured to determine, based on output signals from one or more sensor 150, whether subject 105 is engaged with interface appliance 160 and/or whether subject 105 is ready to receive pressurized flow of breathable gas. For example, in embodiments where interface appliance 160 is a mouthpiece, delivery triggering module 111 may determine whether subject 105 has engaged with the interface appliance based on whether subject 105 has received the mouthpiece in his/her mouth. In such embodiments, delivery triggering module 111 may determine whether subject 105 is ready to receive pressurized flow of breathable gas when there is negative pressure at the mouthpiece resulting from diaphragmatic movements by subject 105.

Additionally, it is contemplated, in accordance with the present disclosure, that readiness of the subject may be indicated merely by subject 105 making a contact with interface appliance 160. For example, in one embodiment, for a subject suffering from a neuromuscular disorder, a therapy regimen may prescribe that delivery of pressurized flow of breathable gas be initiated when subject 105 connects his/her mouth to interface appliance 160. In such embodiments, an inspiratory effort may not be considered a necessary condition for initiating the delivery of pressurized flow of breathable gas. Accordingly, delivery triggering module 111, in such embodiments, may determine that the subject is ready to receive the pressurized flow of breathable gas when subject 105 engages with interface appliance 160.

Control module 112 may be configured to control pressure generator 140 to adjust one or more gas parameters, described elsewhere herein, of the pressurized flow of breathable gas. Control may be in accordance with a prescribed respiratory therapy regimen, one or more algorithms that control adjustments and/or changes in the pressurized flow of breathable gas over time, operational settings, and/or other factors. For example, subject 105 or user 195 may provide one or more settings that correspond to one or more particular pressure levels, one or more modes of operation, and/or one or more preferences related to the operation of pressure generator 140. Control module 112 may be configured to control pressure generator 140 to provide the pressurized flow of breathable gas. Control module 112 may be configured to control pressure generator 140 such that one or more gas parameters of the pressurized flow of breathable gas are varied over time in accordance with a respiratory therapy regimen.

Control module 112 may be configured to initiate and/or terminate delivery of the pressurized flow of breathable gas to the airway of subject 105 through interface appliance 160 by suitably adjusting parameters of the pressurized flow of breathable gas from pressure generator 140. Control module 112 may communicate with delivery triggering module 111 prior to initiating and/or terminating delivery of pressurized flow of breathable gas to the airway of subject 105. For example, when delivery triggering module 111 determines that subject 105 is engaged and/or ready to receive pressurized flow of breathable gas, delivery triggering module 111 may communicate control module 112 to initiate the pressurized flow of breathable gas. Control module 112 may then communicate with pressure generator 140 to adjust flow parameters such that delivery of pressurized flow of breathable gas is initiated. Likewise, when delivery triggering module 111 determines that subject 105 has lost contact with or disengaged from interface appliance 160, control module 112 may communicate with pressure generator 140 to terminate the pressurized flow of breathable gas to subject 105.

In some embodiments, a schedule for delivery of pressurized flow of breathable gas is prescribed according to a therapy regimen by a user (e.g. user 195) via user interface 120. For example, user 195 may prescribe automatic delivery of a particular volume of breathable gas through interface appliance 160 if subject 105 has not initiated a delivery for a prescribed threshold time. In such embodiments, control module 112 may automatically initiate delivery of pressurized flow of breathable gas to interface device 160 when time between two successive deliveries has exceeded the prescribed threshold time. In such embodiments, control module 112 may not wait for delivery triggering module 111 to determine whether the subject is ready to receive pressurized flow of breathable gas.

In some embodiments, user 195 may program control module 112 via user interface 120 to initiate an alarm signal if subject 105 fails to receive pressurized flow of breathable gas for longer than a prescribed amount of time. Alarm signals in various embodiments may include auditory, visual, textual, tactile, and/or other sensory signals.

In some other embodiments, depending on a condition of the subject, user 195 may program control module 112 to place system 100 in a "standby" mode when subject 105 is not engaged with interface appliance 160. In such embodiments, system 100 may not initiate an alarm signal merely when the subject is disconnected from the system. In "standby" mode, control module 112 is configured to wait indefinitely for subject 105 to engage with interface 160 to initiate delivery of pressurized flow of breathable gas. In such embodiments no alarms may be initiated if subject 105 does not engage with interface appliance 160. In such embodiments, system 100 is used by the subject only when the subject needs assistance in respiration.

Parameters determined by other modules of system 100, received through sensors 150, and/or obtained through other ways may be used by control module 112, e.g. in a feedback manner, to adjust one or more operational settings and/or modes. Alternatively, and/or simultaneously, signals and/or information obtained through user interface 120 may be used by control module 112. Control module 112 may be configured to time its operations relative to transitional moments in the breathing cycle of a subject, over multiple breath cycles, and/or in any other relation to, e.g., any determinations by any of the computer program modules of system 100.

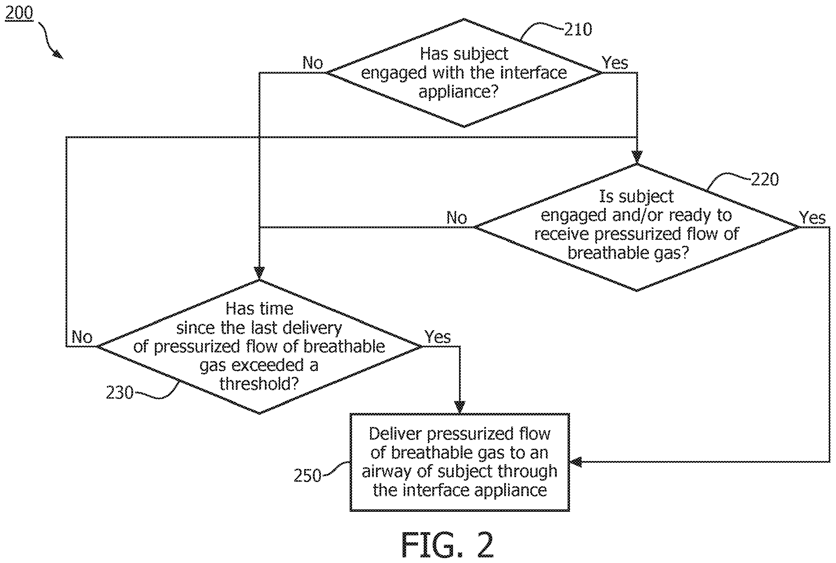

FIG. 2 illustrates a method 200 for providing respiratory support to a subject. Operations of the method 200 presented herein are indented to be illustrative. In certain embodiments, method 200 may be accomplished with one or more additional operations not described, and/or without one or more operations discussed. Additionally, the order in which the operations of method 200 are illustrated in FIG. 2 and described herein are not intended to be limiting.

In certain embodiments, method 200 may be implemented in one or more processing devices (e.g., a digital processor, an analog processor, a digital circuit designed to process information, an analog circuit designed to process information, a state machine, and/or other mechanisms for electronically processing information). The one or more processing devices may include one or more devices executing some or all of the operations of method 200 in response to instructions stored electronically on an electronic storage medium. The one or more processing devices may include one or more devices configured through hardware, firmware, and/or software to be specifically designed for execution of one or more of the operations of method 200.

At an operation 210, it is determined whether the subject has engaged with the interface appliance. In some embodiments, operation 210 is performed by one or more of the computer program modules described elsewhere herein. The determination may be based on output signals generated by one or more sensors 150 (shown in FIG. 1 and described herein) of the ventilation system.

If the subject has engaged with the interface appliance (YES after operation 210), at operation 220, it is determined whether the subject is engaged and/or ready to receive pressurized flow of breathable gas. In some embodiments, operation 220 is performed by delivery triggering module 111 (shown in FIG. 1 and described herein).

If the subject has not engaged with the interface appliance (NO after operation 210), at operation 230, it is determined whether time since the last delivery of pressurized flow of breathable gas has exceeded a threshold as prescribed by a therapy regimen. Operation 230 may be performed, in various embodiments, by processor 110 (shown in FIG. 1 and described herein) via one or more of the computer program modules described herein.

If the time since the last delivery of pressurized flow of breathable gas has not exceeded a threshold (NO after operation 220), at operation 220, it is determined whether the subject is ready to receive pressurized flow of breathable gas.

At operation 250, pressurized flow of breathable gas is delivered to an airway of the subject through interface appliance 160 upon determination that the subject is ready to receive pressurized flow of breathable gas (YES after operation 220), or upon determination that time since last delivery of pressurized flow of breathable gas has exceeded a threshold prescribed by a therapy regimen (YES after operation 230. In some embodiments, operation 250 is performed by control module 112 (shown in FIG. 1 and described herein) via pressure generator 140 (shown in FIG. 1 and described herein) through interface appliance 160.

In the claims, any reference signs placed between parentheses shall not be construed as limiting the claim. The word "comprising" or "including" does not exclude the presence of elements or steps other than those listed in a claim. In a device claim enumerating several means, several of these means may be embodied by one and the same item of hardware. The word "a" or "an" preceding an element does not exclude the presence of a plurality of such elements. In any device claim enumerating several means, several of these means may be embodied by one and the same item of hardware. The mere fact that certain elements are recited in mutually different dependent claims does not indicate that these elements cannot be used in combination.

Although this description includes details for the purpose of illustration based on what is currently considered to be the most practical and preferred embodiments, it is to be understood that such detail is solely for that purpose and that the disclosure is not limited to the disclosed embodiments, but, on the contrary, is intended to cover modifications and equivalent arrangements that are within the scope of the appended claims. For example, it is to be understood that, to the extent possible, one or more features of any embodiment are contemplated to be combined with one or more features of any other embodiment.

## Claims

1. A ventilation system (100) configured to provide respiratory support by intermittently delivering pressurized flow of breathable gas to a subject, the system comprising:
a pressure generator (140) configured to generate pressurized flow of breathable gas for delivery to an airway of the subject;
an interface appliance (160) in the form of a mouthpiece configured to be at least partially and releasably received into an airway orifice of the subject, the interface appliance further configured to deliver the pressurized flow of breathable gas generated by the pressure generator to the airway of the subject;
one or more sensors (150) configured to generate output signals conveying information indicating whether the subject has engaged with the interface appliance and is ready to receive pressurized flow of breathable gas through the interface appliance, wherein the one or more sensors are configured to provide an indication that the subject has received the interface appliance in his/her mouth and the one or more sensors are configured to provide an indication that the subject has made an inspiratory effort; and
one or more processors (110) configured to execute one or more computer program modules, the computer program modules comprising:
a delivery triggering module (111) configured to determine whether the subject has engaged with the interface appliance based on whether the subject has received the interface appliance in his/her mouth and configured to determine whether the subject is ready to receive the pressurized flow of breathable gas through the indication that the subject has made the inspiratory effort when there is movement of his/her mouth and/or tongue or when there is negative pressure at the interface appliance resulting from a diaphragmatic movement; and
a control module (112) configured to initiate and/or terminate delivery of the pressurized flow of breathable gas to the airway of the subject through the interface appliance based on a determination by the delivery triggering module as to whether the subject is ready to receive the pressurized flow of breathable gas, the control module further configured to control the pressure generator to adjust one or more parameters of the pressurized flow of breathable gas based on a prescribed therapy regimen designed to ventilate the subject.

2. The ventilation system of claim 1, wherein the control module is configured to initiate delivery of the pressurized flow of breathable gas earlier of the subject making an inspiratory effort, or two consecutive deliveries exceeding a threshold time specified by the therapy regimen.

3. The ventilation system of claim 1, wherein the control module is configured to adjust the one or more parameters of the pressurized flow of breathable gas such that the interface appliance delivers a prescribed volume or a prescribed pressure of the breathable gas to the airway of the subject.

## Patentansprüche

1. Ein Beatmungssystem (100) das Atmungsunterstützung bietet, indem es einer Person periodisch einen druckbeaufschlagten Atemluftstrom zuleitet, wobei das System aus Folgendem besteht:
einem Druckgenerator (140), der den Atemwegen einer Person periodisch einen druckbeaufschlagten Atemluftstrom zuleitet;
ein Schnittstellengerät (160) in Form eines Mundstücks, das zumindest teilweise in eine Atemwegsöffnung der Person eingeführt und wieder entnommen werden kann, wobei das Schnittstellengerät zudem so konfiguriert ist, dass der vom Druckgenerator erzeugte druckbeaufschlagte Atemluftstrom in die Atemwege der Person geleitet wird;
mindestens einem Sensor (150), der Ausgangssignale generiert, die angeben, ob die Person das Schnittstellengerät eingeführt hat und bereit ist, einen druckbeaufschlagten Atemluftstrom über das Schnittstellengerät zu erhalten, wobei ein Sensor angeben kann, ob die Person das Schnittstellengerät im Mund eingeführt hat, während ein anderer Sensor angeben kann, ob die Person Atemanstrengungen unternommen hat; und
mindestens einem Prozessor (110), der mindestens ein Computerprogrammmodul ausführt, wobei die Computerprogrammmodule Folgendes umfassen:
ein Modul zum Auslösen der Versorgung (111), das ermittelt, ob die Person das Schnittstellengerät im Mund eingeführt hat, und ob sie anhand des Signals in Bezug auf die Atemanstrengungen bereit ist, einen druckbeaufschlagten Atemluftstrom zu erhalten, wobei dieses Signal aufgrund von Mund- und/oder Zungenbewegungen sowie aufgrund eines Unterdrucks am Schnittstellengerät durch Bewegungen des Zwerchfells ausgelöst werden kann; und
ein Steuerungsmodul (112) das die Zufuhr des druckbeaufschlagten Atemluftstroms über das Schnittstellengerät zu den Atemwegen der Person abhängig davon auslöst und/oder beendet, ob das Modul zum Auslösen der Versorgung erkannt hat, dass die Person bereit ist, den druckbeaufschlagten Atemluftstrom zu erhalten, wobei das Steuerungsmodul zudem den Druckgenerator steuert und diesen anhand von Parametern für den druckbeaufschlagten Atemluftstrom einstellt, die auf der für die Beatmung verschriebenen Behandlung beruhen.

2. Das Beatmungssystem gemäß Anspruch 1, wobei das Steuerungsmodul die Zufuhr des druckbeaufschlagten Atemluftstroms bereits früher auslöst, wenn die Person Atemanstrengungen unternimmt. Zudem kann die Zufuhr zweimal hintereinander erfolgen, wenn ein im Rahmen der Behandlung vorgegebener Zeitschwellenwert überschritten wurde.

3. Das Beatmungssystem gemäß Anspruch 1, wobei das Steuerungsmodul die Parameter für den druckbeaufschlagten Atemluftstrom so anpasst, dass das Schnittstellengerät eine vordefinierte Menge oder einen vordefinierten Druck für den druckbeaufschlagten Atemluftstrom in die Atemwege der Person leitet.

## Revendications

1. Système de ventilation (100) conçu pour fournir une aide respiratoire en délivrant de manière intermittente un flux sous pression de gaz respirable à un sujet, ledit système comprenant :
un générateur de pression (140) conçu pour générer un flux sous pression de gaz respirable destiné à être administré à une voie aérienne du sujet ;
un appareil d'interface (160) sous la forme d'un embout buccal conçu pour être reçu, au moins partiellement et de manière amovible, dans un orifice de la voie aérienne du sujet, ledit appareil d'interface étant en outre conçu pour administrer le flux sous pression de gaz respirable généré par le générateur de pression à la voie aérienne du sujet ;
au moins un capteur (150) conçu pour générer des signaux de sortie véhiculant des informations indiquant si le sujet s'est engagé avec l'appareil d'interface et si le sujet est prêt à recevoir un flux sous pression de gaz respirable à travers l'appareil d'interface, dans lequel l'au moins un capteur est conçu pour fournir une indication que le sujet a reçu l'appareil d'interface dans sa bouche et que l'au moins un capteur est conçu pour fournir une indication que le sujet a effectué un effort inspiratoire ; et
au moins un processeur (110) conçu pour exécuter au moins un module de programme informatique, les modules de programme informatique comprenant :
un module de déclenchement d'administration (111) conçu pour déterminer si le sujet s'est engagé avec l'appareil d'interface en fonction du fait que le sujet a reçu l'appareil d'interface dans sa bouche et conçu pour déterminer si le sujet est prêt à recevoir le flux sous pression de gaz respirable par l'indication que le sujet a fait l'effort inspiratoire lorsqu'il y a un mouvement de sa bouche et/ou de sa langue ou lorsqu'il y a une pression négative au niveau de l'appareil d'interface résultant d'un mouvement diaphragmatique ; et
un module de commande (112) conçu pour initier et/ou terminer l'administration du flux sous pression de gaz respirable à la voie aérienne du sujet à travers l'appareil d'interface en fonction d'une détermination par le module de déclenchement d'administration si le sujet est prêt à recevoir le flux sous pression de gaz respirable, ledit module de commande étant en outre conçu pour commander le générateur de pression afin d'ajuster au moins un paramètre du flux sous pression de gaz respirable en fonction d'un schéma thérapeutique prescrit conçu pour ventiler le sujet.

2. Système de ventilation selon la revendication 1, dans lequel le module de commande est conçu pour initier l'administration du flux sous pression de gaz respirable avant que le sujet fasse un effort inspiratoire, ou deux administrations consécutives dépassant un temps seuil spécifié par le schéma thérapeutique.

3. Système de ventilation selon la revendication 1, dans lequel le module de commande est conçu pour ajuster l'au moins un paramètre du flux sous pression de gaz respirable de telle sorte que l'appareil d'interface administre un volume prescrit ou une pression prescrite du gaz respirable à la voie aérienne du sujet.
